(19) Europäisches Patentamt
European Patent Office
Office européen des brevets

(11) EP 3 476 950 A1

(12) EUROPEAN PATENT APPLICATION

(43) Date of publication:
01.05.2019 Bulletin 2019/18

(51) Int Cl.:
*C12Q 1/6886* (2018.01)

(21) Application number: 17460065.0

(22) Date of filing: 31.10.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: Centrum Onkologii - Instytut Im. Marii Sklodowskiej-Curie
02-034 Warszawa (PL)

(72) Inventors:
• KOWALEWSKA, Magdalena
03-937 Warszawa (PL)
• ZALEWSKI, Kamil
01-134 Warszawa (PL)

(74) Representative: Skrzynska-Majewska, Izabela
LDS Lazewski Depo & Partners
Okopowa 58/72
01-042 Warszawa (PL)

(54) MICRORNA-BASED METHODS AND COMPOSITIONS FOR THE DIAGNOSIS, PROGNOSIS AND TREATMENT OF VULVAR CARCINOMA AND VULVAR INTRAEPITHELIAL LESIONS

(57) The subject of the invention is a method of assessing whether a subject has, or is at risk of developing vulvar squamous cell carcinoma (VSCC) or vulvar squamous intraepithelial lesions (VIN), comprising the steps of:
- measuring the expression level of at least one biomarker in a sample of the test subject, wherein the biomarkers are selected from a group comprising miR-222-3p, miR-23b-3p, miR-431-5p and miR-630 or functional variants thereof,
- comparing said expression level with reference expression level,
- wherein the comparison of said expression level of at least one said biomarker to said reference expression level is indicative to determine whether the subject has, or is at risk of developing vulvar squamous cell carcinoma or vulvar squamous intraepithelial lesions.

EP 3 476 950 A1

**Description**

**Filed of disclosure**

This invention relates generally to the field of molecular biology and oncology. Certain aspects of the invention include application in diagnosis, prognosis follow-up and therapy of vulvar carcinoma and related disorders. The present disclosure provides methods pertaining to the identification of genes and gene regulators that modulate certain biological pathways in vulvar disorders; vulvar carcinoma (VC) and its precursors; vulvar squamous intraepithelial lesions (VIN).

**Background of the invention**

Vulvar carcinoma (VC) is a genital malignancy with age-standardized incidence rates ranging worldwide between 0.5 and 1.5 per 100,000. The current ASR in Poland is 1.13; 515 new cases of VC were diagnosed and 267 VC-related deaths were recorded in 2014 (age-standardized mortality rate 0.5) [Wojciechowska et al.]. It is widely recognized that vulvar squamous cell carcinoma (VSCC) develops on the basis of vulvar squamous intraepithelial lesions (VIN). In the classification adopted in 2015 by the International Society for the Study of Vulvar Disease (ISSVD), vulvar precancerous lesions are divided into two types, differing in their pathogenesis and clinical significance: high grade-squamous intraepithelial lesion (HSIL) previously classified as VIN usual type (uVIN) and differentiated vulvar intraepithelial neoplasia (dVIN) [Bornstein J et al.]. The risk of progression from uVIN to VSCC is low and ranges from 3 to 16%, while for dVIN it reaches 32,8%. In the United States, between 1973 and 2000, the incidence of VIN increased by 411% against 20% for invasive cancers [Akerman G et al.]. Vulvar squamous cell carcinoma (VSCC) is the most common histological type of VC, representing approximately 90 % of lesions. Five-year survival rate for VC patients is approximately 90% for early stages and 20% for advanced stages of the disease (http://seer.cancer.gov/statfacts/html/vulva.html).

Vulvar cancer diagnosis

Most VC and VIN are located in the labia majora, but other possible sites include the labia minora, clitoris, mons, or perineum. In patients with HPV-negative tumours, VC often presents as a single mass or ulcer on the labia majora or minora. In HPV-positive tumours, multifocal lesions and concurrent cervical neoplasia are more common.

There is no specific screening method to detect VC and its precursor lesions. Although many cases may be asymptomatic, pruritus and pain, irritation is a common symptom; vulvar bleeding or discharge may also occur. Most patients present with early-stage (i.e. localized) disease. If symptoms are suspicious for VC, the diagnosis must be primarily based on the clinical work-up. The following methods are used to identify precancerous lesions and carcinomas:

- clinical examination, consisting of careful inspection and palpation of the area including the inguinal region;

- vulvoscopy with the application of acetic acid.

All suspicious lesions must be individually biopsied and examined histologically as cancer may be seen in areas with multifocal vulvar intraepithelial neoplasia. The biopsy should be taken from the areas of the lesion that appear most abnormal. If multiple abnormal areas are present, then multiple biopsies should be taken to "map" all potential sites of vulvar pathology.

There are several diagnostic and differential diagnostic considerations that come into play in the pathological evaluation of VC in general, and its precursor lesions in particular. Thorough sampling of VIN is important, because 3.2-18.8% of biopsies can have unsuspected stromal invasion and be finally diagnosed as cancer. Also the interobserver variability amongst pathologists for assessing invasion is suboptimal ranging from 0.24 for diagnosing invasion and 0.51 for measuring depth of invasion. Also pathologic evaluation may not be possible for specimens taken from areas of extensive necrosis.

No molecular tests nor markers allowing precise and non-invasive diagnosis and/or differentiation of malignant and premalignant lesions of the vulva have been developed to date.

To our knowledge, no one has, so far, conducted studies aiming at the identification of diagnostic or prognostic markers among the circulating microRNAs in VSCC and VIN. The identified microRNAs, demonstrating changes in blood levels likely related to prognosis, may provide basis for the development of a non-invasive test. Such test should allow diagnosis of VIN lesions and its differentiation from other benign conditions without malignant potential, early monitoring of the disease in patients with VIN, more precise VSCC staging, preoperative prognosis of lymph node status and ultimately serve as a prognostic and predictive marker for monitoring efficacy of therapy in VSCC patients.

The constantly increasing number of new VIN and VC cases and unsatisfactory treatment outcomes are a significant societal problem. Therefore, there is a great need to develop an effective, minimally invasive test that would allow to stratify patients with VIN and VSCC and to determine the risk of progression from VIN to VSCC.

**Summary of the invention**

It has now surprisingly been found that a biological test (a biomarker) measured/assessed in the blood could considerably contribute to the improvement of treatment outcomes in VIN and VSCC, as such test could be used as a screening method allowing to identify VIN patients at the high-risk of disease progression to VSCC.

The object of the invention is a combined signature consisting of microRNA determined in plasma that serves to indicate which individuals among individuals with VIN are more likely to have VSCC and, at the same time, serves to rule out the presence of VSCC in the others.

The combined signature is based, inter alia, on the identification of specific miRNAs that have altered expression levels in the blood as well as on the identification of specific miRNAs with stable expression, i.e. normalizers (reference molecules).

The object of the invention is also treatment of VIN and VSCC, which includes miRNA mimics or inhibitors for use in the treatment of VIN or VSCC, and a pharmaceutical composition for treating vulvar disorders.

The invention also provides a method identifying one or more biomarkers for VIN and VSCC.

A kit for performing the method of assessing whether a subject has or is at risk of developing VSCC or VIN is also the subject of the invention.

**Brief description of the drawing**

Fig.1. Expression levels of four selected miRNAs in plasma samples of VIN and VSCC patients.

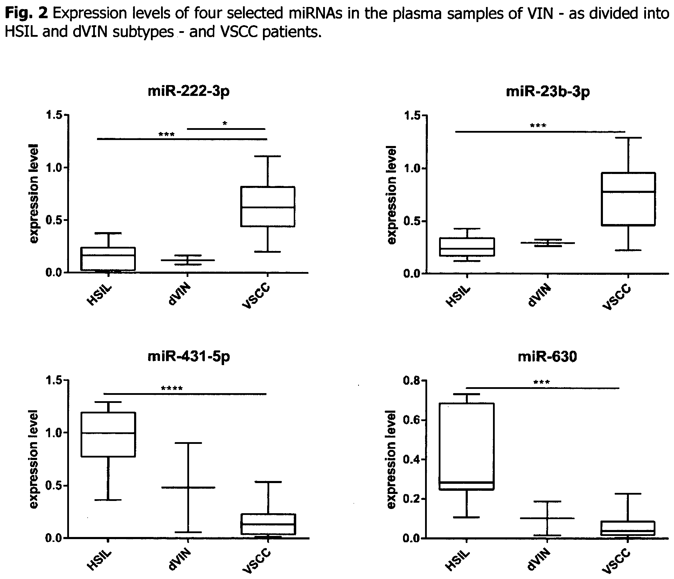

Fig. 2. Expression levels of four selected miRNAs in the plasma samples of VIN - as divided into HSIL and dVIN subtypes - and VSCC patients.

Fig. 3. Receiver operating characteristics (ROC) curve based on the normalized expression levels of miR-222-3p and miR-431-5p.

**Definitions**

The term "microRNA", "miRNA" or "miR" used herein means a non-coding RNA that regulates the translation of the coding mRNA(s) and is between 18 and 25-nucleotide long. These molecules are generally considered to be negative regulators of gene expression [Guo H et al.], yet they may also function as inducers of translation [Vasudevan S. et al.]. miRNAs are involved in a variety of both normal and pathological biological processes.

Examples and sequences of 1881 Homo sapiens miRNAs have been identified and are found in the miRNA database known as miRbase (http://www.mirbase.org/cgi-bin/mirna_summary.pl?org=hsa). MicroRNAs are named using the "miR" prefix and a unique identifying number (e.g., miR-1, miR-2, ... miR-89, etc.). The names/identifiers in the database are of the form such as hsa-mir-121. The first three letters signify the organism. The mature miRNA is designated miR-121 in the database and in much of the literature, whilst mir-121 refers to the miRNA gene and also to the predicted stem-loop portion of the primary transcript. Distinct precursor sequences and genomic loci that express identical mature sequences get names of the form such as hsa-mir-121-1 and hsa-mir-121-2. Lettered suffixes denote closely related mature sequences - for example hsa-miR-121a and hsa-miR-121b that would be expressed from precursors hsa-mir-121a and hsa-mir-121b, respectively. miRNA cloning studies sometimes identify two ~22nt sequences miRNAs which originate from the same predicted precursor. When the relative abundancies clearly indicate which is the predominantly expressed miRNA, the mature sequences are assigned names of the form miR-56 (the predominant product) and miR-56* (from the opposite arm of the precursor). When the data are not sufficient to determine which sequence is the predominant one, names like miR-142-5p (from the 5' arm) and miR-142-3p (from the 3' arm). Criteria and conventions for miRNA identification and naming are described by [V. Ambros et al. RNA 2003, 9(3):277-279].

The term "VSCC" provided herein refers to vulvar squamous cell carcinoma. VSCC refers to any primary or secondary non-neoplastic malignant condition affects squamous cell epithelia of the vulva. VSCC can be of any subtype, e.g., but not limited to warty, basaloid, keratinizing and non-keratinizing type. Variants of VSCC are classified based upon the microscopic findings, when examined by a pathologist under the microscope [WHO Classification of Tumours of Female Reproductive Organs WHO/IARC Classification of Tumours, 4th Edition, Volume 6, Year: 2014. Edited by Kurman RJ, Carcangiu ML, Herrington CS, Young RH. ISBN-13 978-92-832-2435-8].

The term "VIN" provided herein refers to vulvar intraepithelial neoplasia. VIN denotes a squamous intraepithelial lesion of the vulva that shows dysplasia with varying degrees of atypia. VIN comprises high-grade squamous intraepithelial lesion (HSIL, VIN usual type, uVIN), which is associated with developing into the warty and basaloid type carcinoma (which is associated with oncogenic HPV infection) and differentiated vulvar intraepithelial neoplasia, also known as dVIN, which is associated with vulvar dermatoses such as lichen sclerosus and also with atypia of the squamous epithelium. Therefore, the term VIN as used herein comprises both HSIL and dVIN lesions. The diagnosis of VIN is based on a careful inspection and a targeted biopsy of a visible vulvar lesion.

The terms "measuring the expression level", "measuring the level of miRNA" or "measuring the level of miRNA gene product" are used interchangeably in the art. These terms concern measuring a number of transcript copies produced in gene transcription process. The measuring, as used herein, refers to any method of detection, quantification, identification, visualisation, assessment, profiling, abundance measurement or analysis of miRNA level.

The expression level of miRNA can be measured using any technique that is suitable for detecting miRNA in a biological sample. Suitable techniques for determining miRNA expression level in cells from a biological sample are well known to those of skill in the art. In particular the following techniques may be applied: RT-PCR, quantitative RT-PCR (RT-qPCR), Northern blot analysis [Várallyay E, Burgyán J, Nat Protoc. 2008;3(2):190-6], hybridization to microarrays [Chang-Gong Liu CG at el. Nature Protocols 2008; 3: 563 - 578], miRNA sequencing, in particular next generation sequencing (NGS) [Tam S, de Borja R, Tsao MS and McPherson JD. Laboratory Investigation 2014, 94: 350-358], miRNA detection by microscopy with in situ hybridization (ISH) [Urbanek MA, Nawrocka AU and WJ. Int J Mol Sci. 2015; 16(6): 13259-13286], enzymatic luminescence miRNA assay [Sun Y, Gregory KJ, Chen NG, Golovlev V. Analytical Biochemistry 2012; 429(1):11-17].

The term "Ct" value, as used herein, relates to the value of threshold-cycle obtained in quantitative real-time PCR (qPCR) experiment. The normalized expression level is calculated by the $2^{-\Delta Ct}$ method, quantitating relative microRNA expression.

The term "normalizer", as used herein, relates to reference molecule, which should demonstrate stable expression level, storage stability, extraction, and quantification efficiency equivalent to that of the target of interest. Normalizers are used in order to minimize differences that could arise from sample procurement, stabilization, RNA extraction or quantification methods, or sample-to-sample inconsistencies.

The term "about", as used herein, refers to measurement uncertainty of the specified method and is used to reflect experimental deviations, which may be found for different measurements of the same sample.

An alteration in the level of the miRNA gene may be an increase or a decrease.

Functional variant is understood as a miRNA which varies by one nucleotide.

**Detailed description of the invention**

In the first broad aspect, there is provided herein, a method of assessing whether a subject has, or is at risk of developing a vulvar-related disorder, in particular premalignant or malignant, determining prognosis of a subject with vulvar disorder, and/or treating a vulvar disorder in a subject who has a vulvar related disorder, comprising measuring at least one biomarker in a test sample.

In one embodiment, a method of assessing whether a subject has, or is at risk of developing vulvar squamous cell carcinoma (VSCC) or vulvar squamous intraepithelial lesions (VIN), comprises the steps of:

- measuring the expression level of at least one biomarker in a sample of the test subject, wherein the biomarkers are selected from a group comprising miR-222-3p, miR-23b-3p, miR-431-5p and miR-630 or functional variants thereof;
- comparing said expression levels with reference expression levels,
- wherein the comparison of said expression level of at least one said biomarker to said reference expression level is indicative to determine whether the subject has, or is at risk of developing VSCC or VIN.

The method preferably comprises the steps of:

- measuring the expression level of at least one normalizer in the sample of the test subject;
- normalizing the measured expression level of at least one said biomarker against normalizer to obtain normalized value of the expression level of at least one said biomarker;

and wherein the normalized value of the expression level of at least one said biomarker is used for comparison with normalized reference expression level.

The normalizers are preferably one or more molecules selected from hsa-miR-93-5p, hsa-miR-425-5p, hsa-miR-191-5p or combination thereof, preferably the combination of hsa-miR-93-5p and hsa-miR-425-5p. The measured expression level can be normalized against more than one normalizer and the average normalized value is calculated, or the expression level can be normalized against a combination of reference molecules.

Said reference expression level is one or more values established from one or more biological samples. The samples may be samples obtained from healthy subjects or subjects having or being at risk of developing VSCC or VIN. Reference expression levels may be expressed as reference intervals (RI) [Jones G, Barker A. Clin Biochem Rev. 2008; 29 (Suppl 1): S93-S97]. The current paradigm for pathology reference intervals is for each laboratory to determine its own interval for use with each test offered by the laboratory [Jones GR, Barker A, Tate J, Lim CF and Robertson K. Clin Biochem Rev. 2004; 25(2): 99-104].

In certain embodiments, the sample comprises one or more of serum or blood plasma or whole blood samples. In another embodiments, the sample is blood fractions such as plasma, serum, blood cell fractions or platelets; urine or saliva, swabs from the vulva and vaginal introitus. In preferred embodiment, the sample is blood plasma.

In certain embodiments, at least one biomarker is differentially expressed in the plasma of the subject with tumour (VSCC) and preinvasive lesion (VIN), and is one or more of miR-222-3p, miR-23b-3p, miR-431-5p and miR-630, or variants thereof.

In one embodiment the normalized value for miR-222-3p expression level in a range about 0.26 - 1.1 indicates VSCC, and/or the normalized value for miR-222-3p expression level in a range about 0.0 - 0.25 indicates VIN. Preferably the normalized value for miR-222-3p expression level larger than about 0.25 indicates VSCC. Preferably the normalized value for miR-222-3p expression level lower than about 0.25 indicates VIN.

In one embodiment the normalized value for miR-431-5p expression level in a range about 0.0 - 0.4 indicates VSCC, and/or the normalized value for miR-431-5p expression level in a range about 0.5 - 1.3 indicates VIN. Preferably the normalized value for miR-431-5p expression level lower than about 0.5 indicates VSCC. Preferably the normalized value for miR-431-5p expression level larger than about 0.5 indicates VIN.

In one embodiment the normalized value for miR-630 expression level in a range about 0.0 - 0.15 indicates VSCC, and/or the normalized value for miR-630 expression level in a range about 0.16 - 0.7 indicates VIN. Preferably the normalized value for miR-630 expression level lower than about 0.15 indicates VSCC. Preferably the normalized value for miR-630 expression level larger than about 0.15 indicates VIN.

In one embodiment the normalized value for miR-23b-3p expression level in a range about 0.35 - 1.3 indicates VSCC, and/or the normalized value for miR-23b-3p expression level in a range about 0.1 - 0.34 indicates VIN. Preferably the normalized value for miR-23b-3p expression level larger than about 0.35 indicates VSCC. Preferably the normalized value for miR-23b-3p expression level lower than about 0.35 indicates VIN.

In certain embodiments, the level of the biomarker in the blood sample of VSCC patient is greater or lower than the level of the corresponding biomarker in the blood sample of VIN patient.

In another broad aspect, there is provided herein, a method of screening for one or more biomarkers for VSCC or VIN in a subject, comprising: obtaining a sample of blood plasma from a subject, measuring the expression level of at least one biomarker, wherein the biomarkers are selected from one or more of miR-222-3p, miR-23b-3p, miR-431-5p and miR-630, or functional variants thereof. The expression level of at least one said biomarker is preferably normalized against normalizer present in the sample to obtain the normalized value of the expression level of at least one said biomarker and the normalized value of the expression level is used for further analysis. The normalizers are preferably one or more molecules selected from hsa-miR-93-5p, hsa-miR-425-5p, hsa-miR-191-5p or a combination thereof, preferably the combination of hsa-miR-93-5p and hsa-miR-425-5p.

In another broad aspect, there is provided herein, a method of screening for one or more biomarkers for VSCC or VIN in a subject, comprising: obtaining a sample of blood plasma from a subject, conducting reverse transcription quantitative real-time polymerase chain reaction (RT-qPCR), and quantifying one or more than one biomarkers differentially expressed between blood of VSCC patients and VIN patients, wherein the biomarkers are selected from one or more of miR-222-3p, miR-23b-3p, miR-431-5p and miR-630, or functional variants thereof. The expression level of at least one said biomarker is preferably normalized against normalizer present in the sample to obtain the normalized value of the expression level of at least one said biomarker, and the normalized value of the expression level is used for further analysis. The normalizers are preferably one or more molecules selected from hsa-miR-93-5p, hsa-miR-425-5p, hsa-miR-191-5p or a combination thereof, preferably the combination of hsa-miR-93-5p and hsa-miR-425-5p.

In preferred embodiment, RT-qPCR is used for measuring the expression level of at least one biomarker selected from one or more of miR-222-3p, miR-23b-3p, miR-431-5p and miR-630, or functional variants thereof.

Measuring the expression level of at least one biomarker selected from one or more of miR-222-3p, miR-23b-3p, miR-431-5p and miR-630, or functional variants thereof may be performed using a method comprising northern blotting, hybridization to microarrays, microRNA sequencing (e.g., next generation sequencing), single cell miRNA detection by microscopy with in situ hybridization (ISH), enzymatic luminescence miRNA assay. Furthermore, other methods of microRNA detection known in the art can be used for measuring the expression level of at least one said biomarker.

In another broad aspect, there is provided herein, a method for influencing transcript abundance and/or protein expression of target miR in the blood of the subject in need thereof, comprising deregulating one or more microRNAs selected from one or more of miR-222-3p, miR-23b-3p, miR-431-5p and miR-630, or functional variants thereof.

In certain embodiments, the method includes altering expression of one or more of miR-222-3p, miR-23b-3p, miR-431-5p and miR-630 to inhibit the protein expression of cancer-related genes.

In another broad aspect, there is provided herein, a method for screening for vulvar pathology in a subject in need thereof.

In another broad aspect, there is provided herein, a method for follow-up after treatment for vulvar pathology in a subject in need thereof.

In another broad aspect, there is provided herein, a blood sample microRNA signature for a vulvar related pathology comprising: one or more of: miR-222-3p, miR-23b-3p, miR-431-5p and miR-630, or a functional variants or combinations thereof that are up-regulated or that are down-regulated.

In another broad aspect, there is provided herein, a method for regulating one or more of genes expressed by VSCC or VIN cells, comprising the step of altering expression of miR-222-3p, miR-23b-3p, miR-431-5p and miR-630 in vulvar cells.

In another broad aspect, there is provided herein, use of mimics of miR-431-5p and miR-630, or functional variants or combinations thereof, as a microRNAs targeting at least one gene in vulvar cells and thus inhibiting protein expression of such gene.

In another broad aspect, there is provided herein, use of inhibitors of miR-222-3p and miR-23b-3p, or functional variants or combinations thereof, as a microRNAs targeting at least one gene in vulvar cells and thus increasing protein expression of such gene.

In another broad aspect, there is provided herein, a method of treating vulvar disorders in a subject who has vulvar disorder in which at least one biomarker is down-regulated or up-regulated in the plasma, serum or whole blood of the subject with vulvar disorder, administrating to the subject an effective amount of mimic or inhibitor of at least one biomarker, or a variant or biologically-active fragment thereof, such that proliferation of VSCC or VIN cells in the subject is inhibited, wherein the biomarker is selected from one or more of miR-222-3p, miR-23b-3p, miR-431-5p and miR-630, or functional variants thereof.

In another broad aspect, there is provided herein, a pharmaceutical composition for treating vulvar disorders, comprising at least one isolated biomarker, and a pharmaceutically-acceptable carrier, wherein the biomarker is selected from one or more of miR-222-3p, miR-23b-3p, miR-431-5p and miR-630, or functional variants thereof.

The invention also provides a method of assessing whether a subject has, or is at risk of developing a VIN or VSCC, comprising:

- measuring the expression level of at least one biomarker in a sample of the test subject,
- measuring the expression level of at least one normalizer in the sample of the test subject, wherein the normalizer is one or more molecules selected from the group of hsa-miR-93-5p, hsa-miR-425-5p, hsa-miR-191-5p;
- normalizing the measured expression level of at least one biomarker against normalizer to obtain a normalized value of the expression level of at least one said biomarker,
- comparing the normalized expression level with normalized reference expression level, wherein the comparison of said normalized expression level of at least one said biomarker to said normalized reference expression level is indicative to determine whether the subject has, or is at risk of developing VIN or VSCC,
- wherein said expression level is one or more values established from one or more biological samples.

The invention also provides a method of identifying one or more biomarkers for VIN or VSCC in a subject, comprising the steps of:

- measuring the expression level of at least one candidate biomarker in a sample of the test subject,
- measuring the expression level of at least one normalizer in the sample of the test subject, wherein the normalizer is one or more molecules selected from hsa-miR-93-5p, hsa-miR-425-5p, hsa-miR-191-5p or combination thereof,
- normalizing the measured expression level of at least one said biomarker against normalizer to obtain the normalized value of the expression level of at least one said biomarker,
- comparing the normalized expression level with normalized reference expression level, wherein the comparison of said normalized expression level of at least one said biomarker to said normalized reference expression level is indicative to determine whether the candidate biomarker is a biomarker for VIN or VSCC,
- wherein said reference expression level is one or more values established from one or more biological samples.

The invention also provides a kit for performing a method of assessing whether a subject has, or is at risk of developing VSCC or VIN, comprising primers and probes specific to miRNA sequences of at least one of miR-222-3p, miR-23b-3p, miR-431-5p and miR-630. The term "target nucleotide sequence" or "target nucleotide" as used herein, refers to the polynucleotide sequence that is sought to be detected and/or quantified, i.e. the sequence that is targeted by the microRNA-specific primers and/or probes. The target miRNA nucleotide sequences are amplified and detected, thereby simultaneously detecting the expression levels of miR-222-3p, miR-23b-3p, miR-431-5p and miR-630. Alternatively, the methods may be applied directly on cDNA without the need for reverse transcription.

The kit typically comprises specific primers and probes specific to miRNA sequences of at least one of miR-222-3p, miR-23b-3p, miR-431-5p and miR-630, dNTP, as well as polymerase, reaction buffer and $Mg^{2+}$.

**Examples**

**Identification of reference microRNA**

The blood samples obtained from 44 patients with VIN and VSCC were included in the study. Among seventeen patients treated for VIN between December 2013 and October 2014 enrolled 12 and 5 were diagnosed with HSIL and d-VIN, respectively. The median age of patients treated for VIN was 58,4 years (range 33-79). Overall, twenty seven patients surgically treated for primary VSCC (T1-2, NO-2, M0) at the Maria Sklodowska-Curie Institute - Oncology Center in Warsaw (CO-I) between February 2005 and May 2011 were recruited. No patient received preoperative chemotherapy or radiotherapy. The median age of patients treated for VSCC was 75.4 years (range 54-92). The study was approved by the Independent Ethics Committee of all the institutions, CO-I (No. 44/2002, 16/2015), HCC (No. 15/2014) and WUM (No. 247/2015), and all patients gave their informed consent.

Plasma samples were obtained from patients' blood before their surgical treatment and stored at - 70°C until RNA isolation. Total RNA was isolated from 200 $\mu$l of plasma using miRNeasy Mini Kit (Qiagen) and miRNA was reverse transcribed using miScript II RT kit (Qiagen) according to the manufacturer's protocol.

Expression levels of six candidate reference miRNAs, i.e. hsa-miR-93-5p, hsa-miR-425-5p, hsa-miR-16-5p, hsa-miR- 103a-3p, hsa-miR-191-5p, hsa-miR-423-5p, were analysed by quantitative PCR (RT-qPCR) using the miScript miRNA Arrays (Qiagen) according to the manufacturer's protocol. The microRNA molecules in question served as candidate normalizers. Reactions were performed in 7500 Fast Real-Time PCR System (Applied Biosystems) in a final volume of 25 $\mu$l reaction mix, according to the arrays' manufacturer instructions (Qiagen).

Gene expression data were analysed using geNorm™ algorithm [https://genorm.cmgg.be/] integrated into DataAssist™ Software (Applied Biosystems, Thermo Fisher Scientific, Carlsbad, CA, USA). RefFinder, a web-based application which integrates three most commonly used normalization algorithms (geNorm, Normfinder and BestKeeper), was used for results cross-validation [Xie et al.].The algorithm used allowing to determine the most stable normalizers from a set of tested candidate normalizers in a given sample panel. It calculates a gene stability measure (M) according to formulas described by Vandesompele et al. Genes with the lowest M values (<1.5) have the most stable expression.

Our analysis of miRNA expression data with the algorithm identified hsa-miR-93-5p (M= 1.3276) as the gene that retained the greatest robustness in all the plasma samples analysed, i.e. of both VIN and VSCC patients (Tab. 1, bolded font). We identified hsa-miR-93-5p (M=1. 4481) as the most stable reference miRNA (normalizer) for VIN. In plasma samples of patients with VSCC hsa-miR-425-5p (M=1.017) followed by hsa-miR-93-5p (M=1.044) and hsa-miR-191-5p (M=1.3937) were the most stable reference miRNA (normalizer).

This finding enabled us to avoid the common error of using inadequate normalizers in qPCR analysis of microRNA expression, such as snoRNAs which have variable expression due to their involvement in carcinogenesis [Mannoor et al.].

**Tab.1**. Putative reference miRNAs (normalizers) and their expression stability values in plasma samples of VIN and VSCC patients.

| miRNA | miRBase accession number | Stability values for candidate reference genes in plasma calculated by the geNorm™ program | | |
| --- | --- | --- | --- | --- |
| | | VIN | VSCC | VSCC and VIN |
| hsa-miR-93-5p | MIMAT0000093 | **1.4481** | 1.044 | **1.3276** |
| hsa-miR-425-5p | MIMAT0003393 | 1.6850 | **1.017** | 1.3909 |
| hsa-miR-16-5p | MIMAT0000069 | 1.6182 | 1.4196 | 1.6871 |
| hsa-miR-103a-3p | MIMAT0000101 | 2.1591 | 1,4511 | 1.8671 |

(continued)

| miRNA | miRBase accession number | Stability values for candidate reference genes in plasma calculated by the geNorm™ program | | |
| --- | --- | --- | --- | --- |
| | | VIN | VSCC | VSCC and VIN |
| hsa-miR-191-5p | MIMAT0000440 | 2.5022 | 1.3937 | 2.0446 |
| hsa-miR-423-5p | MIMAT0004748 | 1.8651 | 1.4748 | 1.6876 |

**Identification of biomarker microRNAs**

Next, we evaluated expression level of 20 microRNAs in the plasma samples of 23 patients: 11 with VIN (9 diagnosed with HSIL and 2 with d-VIN) and 12 with VSCC. Briefly, total RNA was isolated from 200 $\mu$l of plasma using miRNeasy Mini Kit (Qiagen) and miRNA was reverse transcribed using miScript II RT kit (Qiagen) according to the manufacturer's protocol. Expression levels of 20 microRNAs listed in Tab. 2, were analysed by quantitative RT-PCR (RT-qPCR) using the miScript miRNA Arrays (Qiagen) according to the manufacturer's protocol. To normalize the results we used the combination of the most stable reference microRNA (normalizer) i.e. hsa-miR-93-5p (miRBase Accession No. MIMAT0000093, having mature sequence CAAAGUGCUGUUCGUGCAGGUAG) and hsa-miR-425-5p (miRBase Accession No. MIMAT0003393, having mature sequence AAUGACACGAUCACUCCCGUUGA) (Table 1, bolded font). Reactions were performed in 7500 Fast Real-Time PCR System (Applied Biosystems) in a final volume of 25 $\mu$l reaction mix, according to the arrays' manufacturer instructions (Qiagen). The collected data were analysed using threshold-cycle (Ct) values for the miRNAs with the SDS 2.1 software (Applied Biosystems). The relative amounts of each miR in the plasma of patients with VIN and VSCC were calculated by the $2^{-\Delta Ct}$ method using DataAssist™ Software (Applied Biosystems). Expression levels for the selected miRs were graphed using GraphPad Prism version 6.07.

**Tab.2.** List of microRNAs surveyed in the plasma samples of VIN and VSCC patients.

| Mature miRNA ID | miRBase Accession No. | Mature sequence |
| --- | --- | --- |
| hsa-miR-630 | MIMAT0003299 | AGUAUUCUGUACCAGGGAAGGU |
| hsa-miR-19b | MIMAT0000074 | UGUGCAAAUCCAUGCAAAACUGA |
| hsa-miR-130b | MIMAT0000691 | CAGUGCAAUGAUGAAAGGGCAU |
| hsa-miR-21 | MIMAT0000076 | UAGCUUAUCAGACUGAUGUUGA |
| hsa-miR-532-5p | MIMAT0002888 | CAUGCCUUGAGUGUAGGACCGU |
| hsa-miR-210 | MIMAT0026475 | AGCCCCUGCCCACCGCACACUG |
| hsa-miR-20a | MIMAT0000075 | UAAAGUGCUUAUAGUGCAGGUAG |
| hsa-miR-222-3p | MIMAT0000279 | AGCUACAUCUGGCUACUGGGU |
| hsa-miR-144 | MIMAT0000436 | UACAGUAUAGAUGAUGUACU |
| hsa-miR-155 | MIMAT0000646 | UUAAUGCUAAUCGUGAUAGGGGU |
| hsa-miR-133a | MIMAT0026478 | AGCUGGUAAAAUGGAACCAAAU |
| hsa-miR-1 | MIMAT0000416 | UGGAAUGUAAAGAAGUAUGUAU |
| hsa-miR-206 | MIMAT0000462 | UGGAAUGUAAGGAAGUGUGUGG |
| hsa-miR-29b | MIMAT0000100 | UAGCACCAUUUGAAAUCAGUGUU |
| hsa-miR-145* | MIMAT0004601 | GGAUUCCUGGAAAUACUGUUCU |
| hsa-miR-7 | MIMAT0000252 | UGGAAGACUAGUGAUUUUGUUGU |
| miR-23b-3p | MIMAT0000418 | AUCACAUUGCCAGGGAUUACC |
| miR-431-5p | MIMAT0001625 | UGUCUUGCAGGCCGUCAUGCA |
| miR-543 | MIMAT0004954 | AAACAUUCGCGGUGCACUUCUU |

(continued)

| Mature miRNA ID | miRBase Accession No. | Mature sequence |
|---|---|---|
| let-7c | MIMAT0000064 | UGAGGUAGUAGGUUGUAUGGUU |

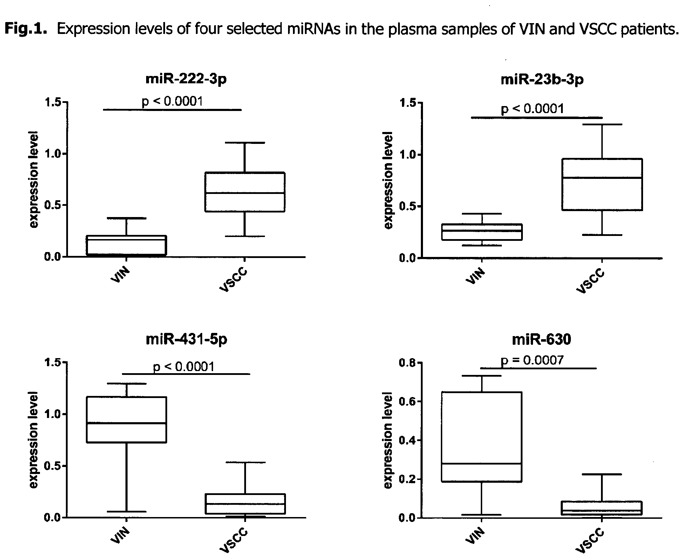

The results pointing at four microRNA molecules which levels were differentiating the tested samples (VSCC *vs* VIN) are depicted in Fig. 1.

The results identify four microRNA molecules that differentiated tested samples (VSCC *vs* VIN) and these differences were statistically significant. The current disclosure reveals that expression level of miR-222-3p, miR-23b-3p, miR-431-5p and miR-630 are circulating biomarkers for differential diagnosis of VIN and VSCC. The levels of miR-222-3p and miR-23b-3p were decreased in the blood of patients with VIN and increased in VSCC, whereas the levels of miR-431-5p and miR-630 were increased in the blood of patients with VIN and decreased in VSCC.

The method of comparison of expression levels of miR-222-3p, miR-23b-3p, miR-431-5p and miR-630 is of high prediction value and is indicative for diagnosis of vulvar squamous cell carcinoma or vulvar squamous intraepithelial lesions, which was confirmed by ROC (receiver operating characteristics). AUCs (areas under the curve) for ROCs were as follows:

-    0.970 for miR-222-3p,
-    0.939 for miR-23b-3p,
-    0.924 for miR-431-5p and
-    0.894 for miR-630.

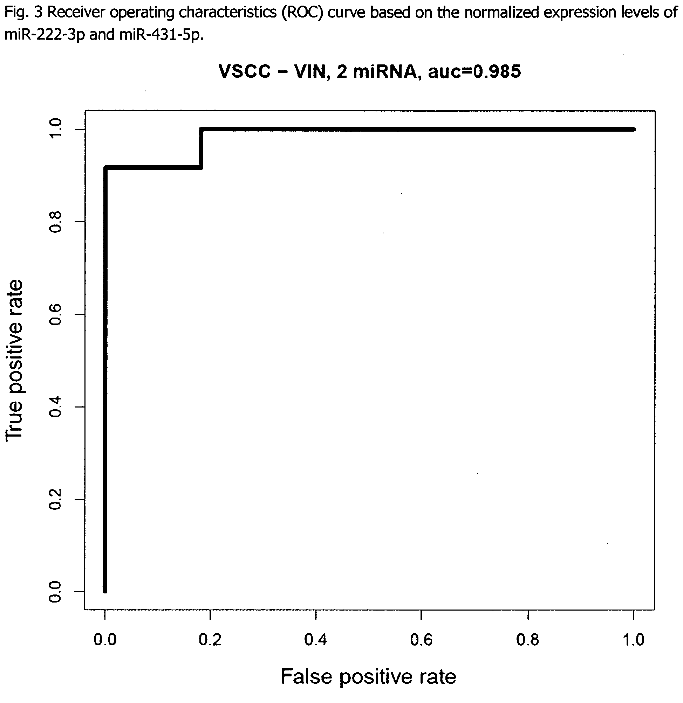

As an example, the normalized expression levels miR-222-3p and miR-431-5p were used simultaneously to estimate the sensitivity and specificity of the sample classifier (Fig. 3). This combination classifier had an AUC of 0.985 and accurately identified patients with VIN and VSCC, thus exhibited excellent performance.

Moreover, our analysis revealed the possibility of building a sample type predictor according to the following formula:

## Predictor Score = (coeff x inverse logit of X) + (coeff x inverse logit of Y)

whereas

coeff is -1.836
X is the value of normalized expression of miR-222-3p
Y is the value of normalized expression of miR-431-5p

The resulting score> 0.5 assigns the sample to the VSCC group, while the score < 0.5 assigns the sample to the VIN group.

Non-patent literature:

1. Wojciechowska U, Didkowska J. Zachorowania i zgony na nowotwory złosliwe w Polsce. Krajowy Rejestr Nowotworów, Centrum Onkologii - Instytut im. Marii Skłodowskiej - Curie

2. Bornstein J, Bogliatto F, Haefner HK, Stockdale CK, Preti M, Bohl TG, Reutter J, Committee IT: The 2015 international society for the study of vulvovaginal disease (issvd) terminology of vulvar squamous intraepithelial lesions. J Low Genit Tract Dis 2016;20:11-14.

3. Ji T, Zheng ZG, Wang FM, Xu U, Li LF, Cheng QH, Guo JF, Ding XF: Differential microrna expression by solexa sequencing in the sera of ovarian cancer patients. Asian Pac J Cancer Prev 2014;15:1739-1743.Vasudevan S. et al.

4. V. Ambros et al. "A uniform system for microRNA annotation". RNA 2003 9(3):277-279.

5. WHO Classification of Tumours of Female Reproductive Organs WHO/IARC Classification of Tumours, 4th Edition, Volume 6, Year: 2014. Edited by Kurman RJ, Carcangiu ML, Herrington CS, Young RH. ISBN-13 978-92-832-2435-8

6. Várallyay E, Burgyán J, Havelda Z. MicroRNA detection by northern blotting using locked nucleic acid probes. Nat Protoc. 2008;3(2):190-6.

7. Chang-Gong Liu, George Adrian Calin, Stefano Volinia & Carlo M Croce MicroRNA expression profiling using microarrays Nature Protocols 3, 563 - 578 (2008)

8. Shirley Tam, Richard de Borja, Ming-Sound Tsao and John D McPherson, Robust global microRNA expression profiling using next-generation sequencing technologies, Laboratory Investigation (2014) 94, 350-358

9. Martyna O. Urbanek, Anna U. Nawrocka,and Wlodzimierz J. Krzyzosiak Small RNA Detection by in Situ Hybridization Methods Int J Mol Sci. 2015 Jun; 16(6): 13259-13286

10. YeSunKalvin J. GregoryNelson G. ChenValGolovlev Rapid and direct microRNA quantification by an enzymatic luminescence assay Analytical Biochemistry Volume 429, Issue 1, 1 October 2012, Pages 11-17

11. Graham Jones, Antony Barker Reference Intervals Clin Biochem Rev. 2008 Aug; 29 (Suppl 1): S93-S97.

12. Graham RD Jones, Antony Barker, Jill Tate, Chen-Fee Lim and Ken Robertson The Case for Common Reference Intervals Clin Biochem Rev. 2004 May; 25(2): 99-104.

13. Xie F, Xiao P, Chen D, et al. miRDeepFinder: a miRNA analysis tool for deep sequencing of plant small RNAs. Plant Mol Biol. Epub ahead of print 31 January 2012. DOI: 10.1007/s11103-012-9885-2

14. Vandesompele J, De Preter K, Pattyn F, et al. Accurate normalization of real-time quantitative RT-PCR data by geometric averaging of multiple internal control genes. Genome Biol 2002; 3: RESEARCH0034.

15. Mannoor K, Liao J, Jiang F.Small nucleolar RNAs in cancer. Biochim Biophys Acta. 2012;1826(1):121-8

**Claims**

1. A method of assessing whether a subject has, or is at risk of developing vulvar squamous cell carcinoma (VSCC) or vulvar squamous intraepithelial lesions (VIN), comprising the steps of:

   - measuring the expression level of at least one biomarker in a sample of the test subject, wherein the biomarkers are selected from a group comprising miR-222-3p, miR-23b-3p, miR-431-5p and miR-630 or functional variants thereof,
   - comparing said expression level with reference expression level,
   - wherein the comparison of said expression level of at least one said biomarker to said reference expression level is indicative to determine whether the subject has, or is at risk of developing vulvar squamous cell carcinoma or vulvar squamous intraepithelial lesions.

2. The method according to claim 1, comprising the steps of:

   - measuring the expression level of at least one normalizer in the sample of the test subject,
   - normalizing the measured expression level of at least one said biomarker against normalizer to obtain a normalized value of the expression level of at least one said biomarker,

   and wherein the normalized value of the expression level of at least one said biomarker is used for comparison with normalized reference expression level.

3. The method according to claim 1 or 2, wherein the normalizers are one or more molecules selected from hsa-miR-93-5p, hsa-miR-425-5p, hsa-miR-191-5p or combination thereof, preferably the combination of hsa-miR-93-5p and hsa-miR-425-5p.

4. The method according to any of the previous claims, wherein the sample is whole blood; blood fraction such as plasma, serum, blood cell fractions or platelets; urine or saliva, swabs from the vulva and vaginal introitus; preferably blood fraction, more preferably blood plasma.

5. The method according to any of the previous claims, wherein said reference expression level is one or more values established from one or more biological samples.

6. The method according to any of the previous claims, wherein the comparison of said expression level of at least one said biomarker to said reference expression level is represented by normalized expression level values indicative for diagnosis of VIN or VSCC.

7. The method according to claim 6, wherein the normalized value for miR-222-3p expression level in a range 0.26 - 1.1 indicates VSCC, and/or the normalized value for miR-222-3p expression level in a range 0.0 - 0.25 indicates VIN.

8. The method according to claim 6, wherein the normalized value for miR-431-5p expression level in a range 0.0 - 0.4 indicates VSCC, and/or the normalized value for miR-431-5p expression level in a range 0.5 - 1.3 indicates VIN.

9. The method according to claim 6, wherein the normalized value for miR-630 expression level in a range 0.0 - 0.15 indicates VSCC, and/or the normalized value for miR-630 expression level in a range 0.16 - 0.7 indicates VIN.

10. The method according to claim 6, wherein the normalized value for miR-23b-3p expression level in a range 0.35 - 1.3 indicates VSCC, and/or the normalized value for miR-23b-3p expression level in a range 0.1 - 0.34 indicates VIN.

11. A miRNA mimic for use as a microRNAs targeting at least one gene in vulvar cells and thus inhibiting protein expression of such gene, wherein miRNA is one or more of miR-431-5p and miR-630 or functional variants thereof.

12. A pharmaceutical composition for treating vulvar disorders, comprising a mimic of at least one biomarker, and a pharmaceutically-acceptable carrier, wherein the biomarker is selected from one or more of miR-431-5p and miR-630 or functional variants thereof.

13. A method of assessing whether a subject has, or is at risk of developing VIN or VSCC, comprising:

    - measuring the expression level of at least one biomarker in a sample of the test subject,
    - measuring the expression level of at least one normalizer in the sample of the test subject, wherein the normalizer is one or more molecules selected from the group of hsa-miR-93-5p, hsa-miR-425-5p, hsa-miR-191-5p or combination thereof,
    - normalizing the measured expression level at least one said biomarker against normalizer to obtain a normalized value of the expression level of at least one said biomarker,
    - comparing the normalized expression level with normalized reference expression level, wherein the comparison of said normalized expression level of at least one said biomarker to said normalized reference expression level is indicative to determine whether the subject has, or is at risk of developing VIN or VSCC,
    - wherein said reference expression level is one or more values established from one or more biological samples.

14. A method of identifying one or more biomarkers for VIN or VSCC in a subject, comprising the steps of:

    - measuring the expression level of at least one candidate biomarker in a sample of the test subject,
    - measuring the expression level of at least one normalizer in the sample of the test subject, wherein the normalizer is one or more molecules selected from the group of hsa-miR-93-5p, hsa-miR-425-5p, hsa-miR-191-5p or combination thereof,
    - normalizing the measured expression level of at least one said biomarker against normalizer to obtain the normalized value of the expression level of at least one said biomarker,
    - comparing the normalized expression level with normalized reference expression level, wherein the comparison of said normalized expression level of at least one said biomarker to said normalized reference expression level is indicative to determine whether the candidate biomarker is a biomarker for VIN or VSCC.
    - wherein said reference expression level is one or more values established from one or more biological samples.

15. A kit for performing a method of claim 1, comprising primers and probes specific to miRNA sequences of at least one of miR-222-3p, miR-23b-3p, miR-431-5p and miR-630.

**Fig.1.** Expression levels of four selected miRNAs in the plasma samples of VIN and VSCC patients.

**Fig. 2** Expression levels of four selected miRNAs in the plasma samples of VIN - as divided into HSIL and dVIN subtypes - and VSCC patients.

Fig. 3 Receiver operating characteristics (ROC) curve based on the normalized expression levels of miR-222-3p and miR-431-5p.

## VSCC – VIN, 2 miRNA, auc=0.985

Europäisches
Patentamt

European
Patent Office

Office européen
des brevets

**EUROPEAN SEARCH REPORT**

Application Number

EP 17 46 0065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIUHUA YANG ET AL: "miRNA expression profile of vulvar squamous cell carcinoma and identification of the oncogenic role of miR-590-5p", ONCOLOGY REPORTS, vol. 35, no. 1, 22 October 2015 (2015-10-22), pages 398-408, XP055463468, ISSN: 1021-335X, DOI: 10.3892/or.2015.4344 | 1,2, 4-10,12 | INV. C12Q1/6886 |
| Y | * page 398 * | 3,13,14 | |
| Y | US 2009/075258 A1 (LATHAM GARY J [US] ET AL) 19 March 2009 (2009-03-19) * page 1, paragraph 17 * | 3,13,14 | |
| X | WO 2013/022786 A2 (UNIV LOUISVILLE RES FOUND [US]; TAYLOR DOUGLAS D [US]; GERCEL-TAYLOR C) 14 February 2013 (2013-02-14) * claims 51-53 * | 15 | |
| X | WO 2016/178519 A1 (KOREA RES INST BIOSCIENCE & BIOTECHNOLOGY [KR]) 10 November 2016 (2016-11-10) * claim 1 * | 11 | TECHNICAL FIELDS SEARCHED (IPC) C12Q |
| X | Beatriz Maia ET AL: "MiR-223-5p works as an oncomiR in vulvar carcinoma by TP63 suppression", Oncotarget, 9 November 2014 (2014-11-09), XP055463216, DOI: 10.18632/oncotarget.10247 Retrieved from the Internet: URL:https://www.ncbi.nlm.nih.gov/pmc/artic les/PMC5226502/pdf/oncotarget-07-49217.pdf | 1,2, 4-10,12 | |
| Y | * page 49220 * * page 49217 * | 3,13,14 | |

-/--

The present search report has been drawn up for all claims

| Place of search | Date of completion of the search | Examiner |
|---|---|---|
| The Hague | 3 April 2018 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 17 46 0065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIEN N. HOANG ET AL: "Squamous precursor lesions of the vulva: current classification and diagnostic challenges", PATHOLOGY., vol. 48, no. 4, 20 July 2017 (2017-07-20), pages 291-302, XP055463543, AU ISSN: 0031-3025, DOI: 10.1016/j.pathol.2016.02.015 * page 1; figure 1 * ----- | 1-15 | |

TECHNICAL FIELDS
SEARCHED    (IPC)

The present search report has been drawn up for all claims

| Place of search | Date of completion of the search | Examiner |
|---|---|---|
| The Hague | 3 April 2018 | Reuter, Uwe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 46 0065

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2018

| Patent document cited in search report | | Publication date | Patent family member(s) | | Publication date |
|---|---|---|---|---|---|
| US 2009075258 | A1 | 19-03-2009 | NONE | | |
| WO 2013022786 | A2 | 14-02-2013 | AU | 2012294628 A1 | 20-03-2014 |
| | | | CA | 2846211 A1 | 14-02-2013 |
| | | | CN | 103842522 A | 04-06-2014 |
| | | | EP | 2739754 A2 | 11-06-2014 |
| | | | US | 2015018227 A1 | 15-01-2015 |
| | | | WO | 2013022786 A2 | 14-02-2013 |
| WO 2016178519 | A1 | 10-11-2016 | KR | 20160131938 A | 16-11-2016 |
| | | | WO | 2016178519 A1 | 10-11-2016 |

EPO FORM P0459

For more details about this annex : see Official Journal of the European Patent Office, No. 12/82

**REFERENCES CITED IN THE DESCRIPTION**

**Non-patent literature cited in the description**

- **V. AMBROS et al.** *RNA,* 2003, vol. 9 (3), 277-279
- WHO Classification of Tumours of Female Reproductive Organs WHO/IARC Classification of Tumours. 2014, vol. 6
- **VÁRALLYAY E ; BURGYÁN J.** *Nat Protoc.,* 2008, vol. 3 (2), 190-6
- **LIU CG.** *Nature Protocols,* 2008, vol. 3, 563-578
- **TAM S ; DE BORJA R ; TSAO MS ; MCPHERSON JD.** *Laboratory Investigation,* 2014, vol. 94, 350-358
- **URBANEK MA ; NAWROCKA AU ; WJ.** *Int J Mol Sci.,* 2015, vol. 16 (6), 13259-13286
- **SUN Y ; GREGORY KJ ; CHEN NG ; GOLOVLEV V.** *Analytical Biochemistry,* 2012, vol. 429 (1), 11-17
- **JONES G ; BARKER A.** *Clin Biochem Rev.,* 2008, vol. 29 (1), S93-S97
- **JONES GR ; BARKER A ; TATE J ; LIM CF ; ROBERTSON K.** *Clin Biochem Rev.,* 2004, vol. 25 (2), 99-104
- **BORNSTEIN J ; BOGLIATTO F ; HAEFNER HK ; STOCKDALE CK ; PRETI M ; BOHL TG ; REUTTER J ; COMMITTEE IT.** The 2015 international society for the study of vulvovaginal disease (issvd) terminology of vulvar squamous intraepithelial lesions. *J Low Genit Tract Dis,* 2016, vol. 20, 11-14
- **JI T ; ZHENG ZG ; WANG FM ; XU U ; LI LF ; CHENG QH ; GUO JF ; DING XF.** Differential microrna expression by solexa sequencing in the sera of ovarian cancer patients. *Asian Pac J Cancer Prev,* 2014, vol. 15, 1739-1743
- **V. AMBROS et al.** A uniform system for microRNA annotation. *RNA,* 2003, vol. 9 (3), 277-279
- **VÁRALLYAY E ; BURGYÁN J ; HAVELDA Z.** MicroRNA detection by northern blotting using locked nucleic acid probes. *Nat Protoc.,* 2008, vol. 3 (2), 190-6

- **CHANG-GONG LIU ; GEORGE ADRIAN CALIN ; STEFANO VOLINIA ; CARLO M CROCE.** MicroRNA expression profiling using microarrays. *Nature Protocols,* 2008, vol. 3, 563-578
- **SHIRLEY TAM ; RICHARD DE BORJA ; MING-SOUND TSAO ; JOHN D MCPHERSON.** Robust global microRNA expression profiling using next-generation sequencing technologies. *Laboratory Investigation,* 2014, vol. 94, 350-358
- **MARTYNA O. URBANEK ; ANNA U. NAWROCKA ; WLODZIMIERZ J.** Krzyzosiak Small RNA Detection by. *Situ Hybridization Methods Int J Mol Sci.,* June 2015, vol. 16 (6), 13259-13286
- **YESUNKALVIN J. ; GREGORYNELSON G. CHEN-VALGOLOVLEV.** Rapid and direct microRNA quantification by an enzymatic luminescence assay. *Analytical Biochemistry,* 01 October 2012, vol. 429 (1), 11-17
- **GRAHAM JONES ; ANTONY BARKER.** Reference Intervals. *Clin Biochem Rev.,* August 2008, vol. 29 (1), S93-S97
- **GRAHAM RD JONES ; ANTONY BARKER ; JILL TATE ; CHEN-FEE LIM ; KEN ROBERTSON.** The Case for Common Reference Intervals. *Clin Biochem Rev.,* May 2004, vol. 25 (2), 99-104
- **XIE F ; XIAO P ; CHEN D et al.** miRDeepFinder: a miRNA analysis tool for deep sequencing of plant small RNAs. *Plant Mol Biol.*
- **VANDESOMPELE J ; DE PRETER K ; PATTYN F et al.** Accurate normalization of real-time quantitative RT-PCR data by geometric averaging of multiple internal control genes. *Genome Biol,* 2002, 3
- **MANNOOR K ; LIAO J ; JIANG F.** Small nucleolar RNAs in cancer. *Biochim Biophys Acta,* 2012, vol. 1826 (1), 121-8